**Europäisches Patentamt**

**European Patent Office**

**Office européen des brevets**

(19)

(11) Publication number : **0 315 648 B1**

(12) # EUROPEAN PATENT SPECIFICATION

(45) Date of publication of patent specification : **01.04.92 Bulletin 92/14**

(51) Int. Cl.[5] : **A61K 7/06**

(21) Application number : **87905890.7**

(22) Date of filing : **02.09.87**

(86) International application number : **PCT/US87/02168**

(87) International publication number : **WO 88/01502 10.03.88 Gazette 88/06**

(54) SEBUM-DISSOLVING NONAQUEOUS MINOXIDIL FORMULATION.

(30) Priority : **05.09.86 US 904146**

(43) Date of publication of application : **17.05.89 Bulletin 89/20**

(45) Publication of the grant of the patent : **01.04.92 Bulletin 92/14**

(84) Designated Contracting States : **AT BE CH DE FR GB IT LI LU NL SE**

(56) References cited :
**WO-A-85/04577**
**US-A- 2 643 375**
**US-A- 4 596 812**

(73) Proprietor : **THE UPJOHN COMPANY**
**301 Henrietta Street**
**Kalamazoo, Michigan 49001 (US)**

(72) Inventor : **HATZENBUHLER, Douglas, A.**
**2726 Bronson Circle**
**Kalamazoo, MI 49008 (US)**
Inventor : **BROWNE, Jeffrey, Edward**
**7504 Thrasher Lane**
**Kalamazoo, MI 49002 (US)**
Inventor : **PENA, Lorraine, Elisabeth**
**1804 Cambridge Drive**
**Kalamazoo, MI 49001 (US)**

(74) Representative : **Perry, Robert Edward et al**
**GILL JENNINGS & EVERY 53-64 Chancery Lane**
**London WC2A 1HN (GB)**

## Description

The present invention relates to a topical composition containing minoxidil which dissolves sebum, the oil surrounding the hair follicle, and provides a means for penetrating the outer skin layer, the stratum corneum.

Minoxidil is a well-known pharmaceutical compound. It is marketed by The Upjohn Company as the active ingredient in Loniten® tablets for the treatment of hypertension. It is also useful in topical compositions for the treatment of baldness. The structure and use of this compound for this purpose, and topical compositions containing it, are described in US-A-4139619 and US-A-4596812. In particular, US-A-4139619 discloses topical minoxidil compositions containing carriers selected from ointments, lotions, pastes, jellies, sprays and aerosols.

Minoxidil has varying degrees of efficacy for hair growth purposes, depending on the patient, the degree of baldness, the dose and the nature of the topical composition. Currently, minoxidil is administered in a topical composition containing propylene glycol, ethanol and water.

WO-A-8504577 discloses compositions used for hair growth which contain a derivative of certain oxadiazolo-pyrimidine-carbamates as active ingredients and, optionally, solubilisers such as glycols or their esters. EP-A-024939 describes topical pharmaceutical compositions containing minoxidil and lipophilic carriers. Cooper, J. Pharm. Sci. 73:1153 (1984) describes means for increasing skin transport of certain pharmaceutical compounds.

The present invention provides a non-aqueous, non-skin-irritative topical hair growth composition as defined in the claims. This composition has improved efficacy.

By minoxidil is meant the 2,4-pyrimidinediamine, 6-(1-piperidinyl)-3-oxide, analogs as well as salts thereof, as described in U.S. Patents 4,139,619, and 4,596,812, which patents are expressly incorporated by reference herein.

Suitable solvents for minoxidil include propylene glycol, 1,3-butylene diol, polyethylene glycol 200 (PEG 200), polyethylene glycol 400 (PEG 400), isopropanol, ethanol, methanol, 1,5 pentane diol, 1,2,6-trihydroxyhexane, 1,7-heptanediol, 1,4 butane diol and N-methylpyrrolidone and related compounds (see, e.g., J. Pharm. Pharmacol. 37:298-304 (1985).

The suitable non-polar solvents according to the invention are silicone oils such as the following volatile silicone oils: Dow Corning - 344 fluid; Dow Corning - 345 fluid; Union Carbide - V.S. 7207; Union Carbide - V.S. 7158; and Union Carbide - V.S. 7349, and the following nonvolatile (or less volatile) silicone oils: Dow Corning - 200 fluids of various viscosities; and Union Carbide - L-45 fluids of various viscosities.

Suitable cosolvent/penetration enhancers include alcohols such as butanol, hexanol, octanol, decanol, dodecanol and oleyl alcohol; amines, such as isopropyl amine, diisopropyl amine, triethyl amine, triethanol amine and ethylene diamine; carboxylic acids, such as oleic acid, linoleic acid and linolenic acid; esters, such as dibutyl sebacate, dibutyl phthalate, butyl benzoate and ethyl caprate; and others, such as AZONE®, N methyl pyrolidone, bile salts and urea. Oleyl alcohol is the preferred cosolvent.

To aid in the miscibility of the components, preferably an additional cosolvent is added to the cosolvent having a polarity between the minoxidil solvent and the non-polar solvent oleyl alcohol. Thus, for oleyl alcohol, the preferred penetration-enhancer and cosolvent, isopropanol is the preferred additional cosolvent making a miscible solution with volatile silicones (e.g. Dow Corning 344 fluid). The isopropanol is used in the range of from 16 to 27% and makes single phase solutions of all mixtures of interest. The volatility of the isopropanol reduces some of the oiliness caused by the oleyl alcohol, since lesser amounts of oleyl alcohol need be used in these formulations to make a miscible solution than were used prior to the addition of isopropanol. Ethanol can also be used as a less chemically "smelling" cosolvent for these vehicles, but ethanol must be present at concentrations 5-10% greater than isopropanol and the resulting vehicle is not as effective in solubilizing sebum.

Sebum is the relatively non-polar material excreted from the sebaceous glands located in the hair follicle. In order to stimulate hair growth, it is desirable to target topical minoxidil formulations to the sebaceous glands. The present composition, which is miscible with human sebum, accomplishes this purpose.

Hildebrand solubility coefficients (HSC) (see Vaughn, J. Soc. Cosmet. Chem. 36:319-333 (Sept/Oct 1985)) are used to characterize a miscible vehicle using a sebum solubilizing agent of low (i.e. non-polar) Hildebrand solubility coefficient in combination with a skin penetration aid with a Hildebrand solubility coefficient intermediate between that of the non-polar sebum solvent and the more polar minoxidil solvent. The resulting vehicle has Hildebrand solubiliy coefficient close to that of human sebum and can completely solubilize the amount of sebum on the scalp. The currently used more polar vehicles for minoxidil cannot solubilize this amount of sebum.

Based upon the composition of synthetic (or artificial) sebum, the Hildebrand (HSC) solubility coefficient for sebum is 7 or 8 cal$^{\frac{1}{2}}$ cm $^{-3/2}$. Minoxidil shows its best solubility in propylene glycol which has an HSC of 14. Miscibility (the ability of two or more liquids to mix in all proportions) is shown on this scale typically when there is a difference of 2 units. Therefore, in order to lower the HSC of the vehicle from that of pure propylene glycol down to 2 of sebum, a solvent with HSC below that of sebum must be chosen. One of the most suitable solvents is volatile silicone oil with a HSC of 5.8-5.9 Since the silicone oils are totally immiscible with the propylene glycol, it is necessary to add a cosolvent to render the two more miscible.

This cosolvent could have only HSC between 6 and 14, however, the midpoint (approx. 10) should require the smallest amount of cosolvent and is thus preferred.

Minoxidil is not well absorbed through the skin in the prior art formulations (e.g., propylene glycol/ethanol/water). Thus, addition of a vehicle component that enhances skin penetration as well as renders the silicone oils and propylene glycol miscible is desired. Most preferable is oleyl alcohol, having an HSC of 9.8. A single phase (i.e. solution) formulation can be prepared from these materials. This vehicle can completely solubilize the sebum levels on the skin whereas previous minoxidil formulations do not dissolve the amounts of sebum reported to be on-the scalp.

For purposes of skin penetration, it is desirable to have less oleyl alcohol in the formulation (e.g., approximately 1:1 ratio of oleyl alcohol to propylene glycol). However, the formulation is not miscible at the 1:1 ratio. A single phase system (at 1:1 oleyl alcohol:propylene glycol) can be prepared by adding some nonvolatile silicone oil (e.g., Dow Corning 200 fluid) and a surfactant (e.g., Union Carbide SILWET L-77).

Further, high concentrations of oleyl alcohol are dermally irritating. Thus, concentrations of oleyl alcohol of from 10 to 40% of the total solution are preferred. It is more preferred for cosmetic acceptability to use less than 20% oleyl alcohol, so that the composition has a less oily "feel".

Preferred proportions of the components are as follows:

Based upon in vitro transdermal data, the concentration of minoxidil should be from 1.0% to 2.5%; the concentration of propylene glycol from 12% to 25%; and the concentration of oleyl alcohol from 6% to 20%. These vehicles give in vitro human skin transport levels of minoxidil that range from equal to the current 2% minoxidil formulation (20% propylene glycol/60% ethanol/20% water) to approximately 10 fold greater transport, as seen by Example 2.

The use of topical minoxidil compositions is well known to the ordinarily skilled physician or dermatologist. This use is also set forth in the above mentioned U.S. Patents 4,139,619 and 4,596,812.

## DESCRIPTION OF THE PREFERRED EMBODIMENTS

The present invention is seen more fully by the Examples below.

## Example 1

The following formulations are prepared according to the procedure below:

## Procedure

(Step 1) Propylene glycol is measured and added to a suitable container. (Step 2) Propylene glycol is heated to 52°-58° using a water bath, and heated for 10-15 min once the temperature has reached the required range. (Step 3) Minoxidil is weighed and added slowly to the heated propylene glycol with rapid mixing. Mixing continues until the minoxidil is completely dissolved (approx. 30-40 min). Temperature is maintained at 52°-58°C using the water bath. (Step 4) The minoxidil-propylene glycol solution is cooled to room temperature (approx. 25°C). (Step 5) Oleyl alcohol is measured and added to the cooled step 4 solution and mixed for 1 min. (Step 6) If procetyl-10 is added, it is added to the step 5 mixture and mixed for 1 min at this point. (Step 7) Dow Corning 344 is added to the above mixture and mixed for 5-10 min until a uniform mixture is obtained.

| Formulation 1 | Minoxidil USP milled (~90 mg/ml propylene glycol to give saturated solution) | |
|---|---|---|
| Propylene Glycol | | 15% |
| Oleyl Alcohol USP | | 15% |
| Dow Corning 344 (volatile silicone oil) | | 70% |

| Formulation 2 | Minoxidil USP milled (~90 mg/ml propylene glycol) | |
|---|---|---|
| Propylene Glycol USP | | 15% |
| Oleyl Alcohol USP | | 30% |
| Procetyl-10( PEG 10 cetyl ether) | | 10% |
| PGE 10 cetyl ether | | |
| Dow Corning 344 | | 45% |

Formulation 3 Minoxidil USP milled (~90 mg/ml propylene glycol)

| | |
|---|---|
| Propylene Glycol USP | 12.5% |
| Oleyl Alcohol USP | 25% |
| Procetyl-10 | 10% |
| Dow Corning 344 | 52.5% |

Example 2

Based on the foregoing specification, and on techniques known in the art, all of the compositions of the invention are prepared. Three representative nonaqueous formulations of minoxidil were prepared and are characterized by their dermal characteristics as follows:

| Vehicle Transport* | Composition (Vol%) | | | |
|---|---|---|---|---|
| | Propylene Glycol | Oleyl Alc. | IPA | Vol. Silicone |
| High (~12X) | 25 | 15 | 27 | 33 |
| Medium (~4X) | 15 | 7.5 | 25 | 52.5 |
| Low (~1.5X) | 12 | 6 | 25 | 57 |

* Vehicle transport is defined as the ratio of the peak (1 hr) transport flux measured for minoxidil through human cadaver skin for the vehicle listed divided by the "standard" reference vehicle (20% propylene glycol/60% ethanol/20% water) peak minoxidil transport measured on a portion of the same piece of skin.

The weight percent of minoxidil in each of these formulations is: 2.3% for the high transport; 1.3% for the medium transport; and 1.1% for the low transport vehicle, while the reference vehicle contains 2.0% minoxidil.

Autoradiographic examination of drug distribution in Macaque monkeys indicates that a formulation containing 20% propylene glycol, 20% oleyl alcohol, 16% isopropanol, and 44% volatile silicone had an approximately sixfold increase in drug delivery into the sebaceous gland in the hair follicle relative to the drug content away from the hair follicle at an equal distance into the skin. The standard formulation had essentially no difference between the amounts in the follicle and outside the follicle. Human in vivo dermal irritation tests show minimal unoccluded dermal irritation for this composition.

Example 3

Using the procedures of the preceding Examples, and techniques known in the art, the following compositions are prepared. (All concentrations are in volume percentages (volume %)).

## TABLE 1

### Nonaqueous Minoxidil Formulations

| Formulation | Conc. Propylene Glycol | Conc. Oleyl Alcohol | Conc. DC 344 Silicone Oils | Other Composition |
|---|---|---|---|---|
| 1 | 25% | 25% | 50% | 0 |
| 2 | 20% | 30% | 50% | 0 |
| 3 | 20% | 20% | 60% | 0 |
| 4 | 20% | 25% | 55% | 0 |
| 5 | 22.5% | 22.5% | 55% | 0 |
| 6 | 15% | 25% | 60% | 0 |
| 7 | 30% | 20% | 50% | 0 |
| 8 | 25% | 15% | 60% | 0 |
| 9 | 25% | 20% | 55% | 0 |
| 10 | 15% | 30% | 45% | 10% Pro-10* |
| 11 | 12.5% | 25% | 57.5% | 5% Pro-10* |
| 12 | 25% | 10% | 65% | 0 |
| 13 | 15% | 7.5% | 77.5% | 0 |

*Pro-10 = Procetyl-10 (propylene cetyl ether) surfactant added to initial emulsions to improve stability.

**Claims**

1. A non-aqueous, non-skin-irritative topical hair growth composition which has a Hildebrand solubility coefficient of 7 to 8, and which comprises:

(a) minoxidil;

(b) a solvent capable of dissolving minoxidil;

(c) a silicone oil having a Hildebrand solubility coefficient of 6 to 14; and

(d) a cosolvent having a polarity between that of the solvent and the silicone oil, and which enhances the delivery of minoxidil through the stratum corneum.

2. A composition according to claim 1, wherein the solvent is propylene glycol, the cosolvent is a mixture of oleyl alcohol and isopropanol, and the silicone oil is volatile.

3. A composition according to claim 2, which comprises 1 to 2.5% v/v minoxidil, 12 to 25% v/v propylene glycol, 6 to 20% v/v oleyl alcohol and 16 to 27% v/v isopropanol.

4. A composition according to claim 2, wherein the relative amounts of the solvent, the cosolvent and the volatile silicone oil are (in % v/v):

| Propylene Glycol | Oleyl Alcohol | Isopropanol | Silicone Oil |
|---|---|---|---|
| 25 | 15 | 27 | 33 |
| 15 | 7.5 | 25 | 52.5 |
| 12 | 6 | 25 | 57 |

5. A composition according to claim 1, wherein the relative amounts of the solvent, the cosolvent, the volatile silicone oil and, optionally, propylene cetyl ether surfactant are (% v/v):

| Propylene Glycol | Oleyl Alcohol | Silicone Oil | Surfactant |
|---|---|---|---|
| 25 | 25 | 50 | 0 |
| 20 | 30 | 50 | 0 |
| 20 | 20 | 60 | 0 |
| 20 | 25 | 55 | 0 |
| 22.5 | 22.5 | 55 | 0 |
| 15 | 25 | 60 | 0 |
| 30 | 20 | 50 | 0 |
| 25 | 15 | 60 | 0 |
| 25 | 20 | 55 | 0 |
| 15 | 30 | 45 | 10 |
| 12.5 | 25 | 57.5 | 5 |
| 25 | 10 | 65 | 0 |
| 15 | 7.5 | 77.5 | 0 |

6. A method for preparing a composition according to any preceding claim, which comprises formulating together the minoxidil, the solvent, the silicone oil, the cosolvent and, if present, the surfactant.

**Patentansprüche**

1. Nicht-wäßrige, nicht-Haut-irritierende, topische Haarwachstumszusammensetzung, welche eine Hildebrand-Löslichkeitskoeffizienten von 7 bis 8 aufweist und welche

(a) Minioxidil;

(b) ein Lösungsmittel, welches Minoxidil lösen kann;

(c) ein Silikonöl, welches einen Hildebrand-Löslichkeitskoeffizienten von 6 bis 14 aufweist, und

(d) ein Colösungsmittel, welches eine Polarität besitzt, welche zwischen jener des Lösungsmittels und jener des Silikonöls liegt und welches die Abgabe von Minoxidil durch die Hornhautschicht beschleunigt, enthält.

2. Zusammensetzung nach Anspruch 1, worin das Lösungsmittel propylenglykol, das Colösungsmittel eine Mischung aus Oleyl-alkohol und Isopropanol ist und das Silikonöl flüchtig ist.

3. Zusammensetzung nach Anspruch 2, welche 1 bis 2,5 Vol.-% Minoxidil, 12 bis 25 Vol.-% propylenglykol, 6 bis 20 Vol.-% Oleylalkohol und 16 bis 27 Vol.-% Isopropanol enthält.

4. Zusammensetzung nach Anspruch 2, worin die relativen Mengen des Lösungsmittels, des Colösungsmittels und des flüchtigen Silikonöls (in Vol.-%)

| Propylen- glykol | Oleyl- alkohol | Isopropanol | Silikonöl |
|---|---|---|---|
| 25 | 15 | 27 | 33 |
| 15 | 7,5 | 25 | 52,5 |
| 12 | 6 | 25 | 57 |

betragen.

5. Zusammensetzung nach Anspruch 1, worin die relativen Mengen des Lösungsmittels, des Colösungsmittels, des flüchtigen Silikonöls und gegebenenfalls des propylencetylether Oberflächenaktivators (in Vol.-%)

| Propylen- glykol | Oleyl- alkohol | Silikonöl | Oberflächen- aktivator |
|---|---|---|---|
| 25 | 25 | 50 | 0 |
| 20 | 30 | 50 | 0 |
| 20 | 20 | 60 | 0 |
| 20 | 25 | 55 | 0 |
| 22.5 | 22.5 | 55 | 0 |
| 15 | 25 | 60 | 0 |
| 30 | 20 | 50 | 0 |
| 25 | 15 | 60 | 0 |
| 25 | 20 | 55 | 0 |
| 15 | 30 | 45 | 10 |
| 12.5 | 25 | 57.5 | 5 |
| 25 | 10 | 65 | 0 |
| 15 | 7.5 | 77.5 | 0 |

betragen.

6. Verfahren zur Herstellung einer Zusammensetzung nach einem der vorhergehenden Ansprüche, wel-

7

ches das Formulieren des Lösungsmittels des Silikonöls des Colösungsmittels, und falls vorhanden, des Oberflächenaktivators mit dem Minoxidil umfaßt.

## Revendications

1. Composition topique non aqueuse, non irritante pour la peau, destinée à la croissance des cheveux, qui possède un coefficient de solubilité d'Hildebrand de 7 à 8 et qui comprend :

(a) du minoxidil ;

(b) un solvant capable de dissoudre le minoxidil ;

(c) une huile de silicone ayant un coefficient de solubilité d'Hildebrand de 6 à 14 ; et

(d) un co-solvant ayant une polarité intermédiaire entre celle du solvant et de l'huile de silicone, et qui accroît l'administration de minoxidil à travers le stratum corneum.

2. Composition suivant la revendication 1, dans laquelle le solvant est le propylène-glycol, le co-solvant est un mélange d'alcool oléylique et d'isopropanol, et l'huile de silicone est volatile.

3. Composition suivant la revendication 2, qui comprend 1 à 2,5% en volume/volume de minoxidil, 12 à 25% en volume/volume de propylène-glycol, 6 à 20% en volume/volume d'alcool oléylique et 16 à 27% en volume/volume d'isopropanol.

4. Composition suivant la revendication 2, dans laquelle les quantités relatives du solvant, du co-solvant et de l'huile de silicone volatile sont (en % en volume/volume) :

| Propylène-glycol | Alcool oléylique | Isopropanol | Huile de silicone |
|---|---|---|---|
| 25 | 15 | 27 | 33 |
| 15 | 7,5 | 25 | 52,5 |
| 12 | 6 | 25 | 57 |

5. Composition suivant la revendication 1, dans laquelle les quantités relatives du solvant, du co-solvant, de l'huile de silicone volatile et, facultativement, du surfactant consistant en éther cétylique de propylène sont (en % en volume/volume) :

| Propylène-glycol | Alcool oléylique | Huile de silicone | Surfactant |
|---|---|---|---|
| 25 | 25 | 50 | 0 |
| 20 | 30 | 50 | 0 |
| 20 | 20 | 60 | 0 |
| 20 | 25 | 55 | 0 |
| 22,5 | 22,5 | 55 | 0 |
| 15 | 25 | 60 | 0 |
| 30 | 20 | 50 | 0 |
| 25 | 15 | 60 | 0 |
| 25 | 20 | 55 | 0 |
| 15 | 30 | 45 | 10 |
| 12,5 | 25 | 57,5 | 5 |
| 25 | 10 | 65 | 0 |
| 15 | 7,5 | 77,5 | 0 |

6. Procédé de préparation d'une composition suivant l'une quelconque des revendications précédentes, qui consiste à formuler ensemble le minoxidil, le solvant, l'huile de silicone, le co-solvant et, s'il est présent, le surfactant.